# EUROPEAN PATENT SPECIFICATION

(11) **EP 4 186 536 B1**
(45) Date of publication and mention of the grant of the patent: **28.01.2026**
(21) Application number: 21845988.1
(22) Date of filing: 23.07.2021
(51) Int. Cl.: A61L 24/04, A61L 31/04, A61L 31/16, A61L 31/14, C08L 71/02, C08L 89/06, C08L 5/08, C08K 5/053

(54) **ANTI-ADHESION POLYMER COMPOSITION**
ANTIHAFTPOLYMERZUSAMMENSETZUNG
COMPOSITION À BASE DE POLYMÈRES ANTI-ADHÉSIVE

(30) Priority: 24.07.2020 KR 20200092274
(43) Date of publication of application: 31.05.2023
(73) Proprietor: CG Bio Co., Ltd., Seoul 04349 (KR)
(72) Inventor: PARK, Cho Hee, Seongnam-si Gyeonggi-do 13585 (KR); YOON, Hong Sun, Yongin-si Gyeonggi-do 17117 (KR); PARK, Hee Jun, Seoul 06366 (KR); MOON, Bo Mi, Seongnam-si Gyeonggi-do 13357 (KR); KIM, Gwi Jae, Seongnam-si Gyeonggi-do 13242 (KR); PARK, Jun Kyu, Seongnam-si Gyeonggi-do 13643 (KR)
(74) Representative: Eisenführ Speiser
(86) International application number: PCT/KR2021/009540
(87) International publication number: WO 2022/019701

(56) References cited:
- WO-A1-00/05974
- KR-A- 20110 025 530
- KR-A- 20130 098 768
- KR-A- 20130 098 768
- KR-A- 20140 140 212
- KR-A- 20170 047 168
- KR-A- 20190 121 630
- KR-B1- 102 189 740

## Description

### [Technical Field]

The present invention relates to an anti-adhesion polymer composition, and more specifically, to an anti-adhesion polymer composition that has excellent adhesion performance so as to be continuously attached successfully to a wound site while effectively exhibiting an anti-adhesion function, not only has antibacterial and hemostatic properties, but also consists of an injectable formulation so as to be suitable for minimally invasive surgery, laparoscopic surgery, and the like, and can carry growth factors.

### [Background Art]

Adhesion refers to a phenomenon in which blood flows out and coagulates during the healing of a wound caused by inflammation, cuts, friction, cuts caused by surgery or the like, and the like and primarily adheres to surrounding organs or tissues, and cells penetrate into the adhered blood and are organized to produce an excessive amount of fibrous tissue, or surrounding organs or tissues that have to be separated from each other adhere to each other. Further, the level of fibrin around a surgical site is increased by an inflammatory reaction occurring at the surgical site, and in this case, when fibrinolysis normally occurs, no adhesion occurs, but when fibrinolysis is suppressed for some reason, an adhesion occurs accordingly.

This adhesion phenomenon causes serious problems, particularly after surgery. The adhesion phenomenon may generally occur after all types of surgery, and for this reason, organs or tissues around the surgical site may adhere to each other during post-surgery recovery, causing serious clinical sequelae. In general, adhesions occur at a frequency of about 67 to 93% after laparotomies, and in some cases, the adhesion is spontaneously degraded, but in most cases, the adhesion remains even after wound healing, causing various sequelae. Sequelae that are caused by adhesions include bowel dysfunctions, bowel obstructions, chronic pain and the like in the case of laparoscopic surgery, and particularly, adhesions after gynecological surgery are known to cause sterility (Eur. J. Surg. 1997, Supplement 577 , 32-39). An anti-adhesion agent prevents adhesions between tissues from occurring after surgery to eliminate the possibility of secondary diseases, and thus is a very important medical product that contributes directly to the patient's safety.

In order to prevent such adhesions, the following methods have been developed and used: 1) a method of minimizing wounds during surgery; 2) a method of using an anti-inflammatory agent or activating tissue with a tissue plasminogen activator in order to prevent fibrogenesis; and 3) a method of using an anti-adhesion barrier.

Among these methods, the use of the anti-adhesion barrier prevents an adhesion between adjacent tissues from being formed during the healing of a tissue wound. This physical anti-adhesion agent should prevent the formation of an adhesion with an adjacent tissue during the healing of the wound of a tissue and simultaneously should be degraded naturally or removed by absorption after a specific period of time, and either a material that is used in the anti-adhesion agent or a degradation product thereof should be harmless to the human body.

As currently commercialized anti-adhesion agents, polyethylene glycol-polypropylene glycol (PEG-PPG), polyethylene oxide (PEO), poly(lactic acid) (PLA), hyaluronic acid, carboxymethylcellulose (CMC), fibrinogen, a calcium chloride solution, dextran, an icodextrin compound, Teflon^{™}, oxidized regenerated cellulose, polyglycan ester, poloxamer and the like have been used.

However, among such anti-adhesion agents, cellulose and dextran are natural polymers, but are not constituents of the living body, and thus are known to cause a foreign body reaction when inserted into the living body. In addition, it is known that due to the absence of a degrading enzyme for these materials in the living body, the degradation of such materials does not occur, and for this reason, such materials should be manipulated so as to be oxidized or hydrolyzed. Meanwhile, an anti-adhesion solution using hyaluronic acid as a main component has been used, but the anti-adhesion function thereof is limited, because hyaluronic acid has an *in vivo* half-life of only 3 days and thus is easily degraded. Among the synthetic polymers, poly(lactic acid) (PLA) has a disadvantage in that it can cause an inflammatory reaction and a foreign body reaction because a degradation product thereof is acidic in nature.

Furthermore, materials used as anti-adhesion agents include a PEG-PPG-PEG block copolymer. The synthetic polymer PEG-PPG-PEG block copolymer is a polymer produced by BASF and is known as a temperature-sensitive material that is present in a solution state at low temperature, but is gelled when the temperature rises (US Patent Nos. 4,188,373; 4,478,822; and 4,474,751). The PEG-PPG-PEG block copolymer is divided into various types according to the ratio of PEG and PPG, and a PEG-PPG-PEG block copolymer known as poloxamer 407 has a polyoxypropylene molecular mass of 4,000 g/mol and a polyoxyethylene (PEG) content of 70%. Further, a PEG-PPG-PEG block copolymer known as poloxamer 188 has a polyoxypropylene molecular mass of 1,800 g/mol and a polyoxyethylene (PEG) content of 80%. The ratio between the PEG moiety and the PPG moiety, the molecular mass and acidity thereof, and additives affect gelation and physical properties.

In addition, currently commercially available anti-adhesion formulations are morphologically divided into a solution type, a film type and a gel type.

First, solution-type anti-adhesion formulations have a disadvantage in that they can flow into other sites before exhibiting the anti-adhesion function or can be degraded too early, and thus do not sufficiently exhibit the anti-adhesion function in many cases.

Second, film-type anti-adhesion formulations include products such as Seprafilm (hyaluronic acid-CMC), Medishield (CMC-PEO), INTERCEED^{™} (oxidized regenerated cellulose), and SurgiWrap (PLA), but it has been reported that these products have problems in that these products do not easily adhere to the surface of internal organs when applied to the internal organs, and are not continuously positioned in a wound site due to the movement of the organs even though they adhere to the internal organs, and in that they are recognized as foreign matter by tissue itself to agglomerate to each other, and thus have an insufficient effect on the anti-adhesion of organs. However, in spite of such disadvantages, these formulations are in a form capable of reliably providing an anti-adhesion barrier that is one of the anti-adhesion principles, and thus there has been no reliable product capable of substituting for these formulations up to date. Such film-type products have been used mainly in gynecological or spinal surgery in a limited way, and the application thereof to minimally invasive surgery, laparoscopic surgery and the like, which are current surgical trends, is greatly limited. Thus, in order to ensure the anti-adhesion function, there is a need for an anti-adhesion agent that has an excellent adhesive property so as to be able to easily and continuously adhere to a wound site caused by surgery, and has an injectable formulation suitable for use in minimally invasive surgery, laparoscopic surgery and the like, which are current surgical trends.

Finally, gel-type anti-adhesion formulations have been developed as injectable formulations in order to overcome the disadvantages of film-type and solution-type formulations. Currently, there are a product using Dextran 70 called Hyskon^{™}, a product using a PEG-PPG-PEG copolymer called Flowgel^{™}, a product using gelatin called ADCON, a product using hyaluronic acid called INTERGEL, a product using hyaluronic acid-carboxymethylcellulose (CMC) called GUARDIX-sol, and the like. Since the time required for wound healing varies depending on the extent of the wound, but is generally about 7 days, in order to expect anti-adhesion effects, anti-adhesion formulations should assist normal regeneration of a wound site for about 7 days, and should prevent the formation of fibrous tissue with tissues adjacent to the wound for about 7 days, after which they should be naturally degraded, absorbed and removed. However, the gel-type anti-adhesion formulations are dissolved and excreted before wound healing and remain in the wound tissue for an insufficient amount of time, and thus do not exhibit sufficient anti-adhesion effects, and non-bio-derived materials such as cellulose (CMC) and dextran have problems such as foreign body reactions *in vivo.*

Korean Patent No. 1082935 relates to a method for preparing a porous sustained-release formulation film for preventing tissue adhesions, and discloses dissolving polylactide in an organic solvent such as methyl alcohol, ethyl alcohol and acetone, preparing a porous film from the solution, and applying an antibiotic and an anti-inflammatory agent to micropores formed on the surface and inside of the film. However, polylactide, a synthetic polymer called polylactic acid (PLA), has disadvantages in that it creates an acidic environment which may be harmful in the living body when degraded in the living body, because a degradation product thereof is acidic in nature, and biocompatibility deteriorates. Furthermore, a film prepared therefrom has a problem in that the use of polylactide in toxic organic solvents such as methyl alcohol, ethyl alcohol and acetone is likely to cause toxicity in vivo when these organic solvents are not completely removed during the preparation process.

Further, Korean Patent No. 1070358 discloses a medical non-woven anti-adhesion film made of a cellulose-based short fiber that is gelled. This patent has a limitation in that, because a natural cellulose or recycled cellulose material, which is a natural polymer but is not a biomaterial, is used, the material may cause a foreign body reaction when inserted *in vivo.* In addition, there is a disadvantage in that, because enzymes that degrade cellulose-based materials are not present *in vivo,* the cellulose-based material is not completely degraded *in vivo* and is not excreted *ex vivo* in the form of degradation products.

Meanwhile, US Patent No. 6,294,202 discloses an anti-adhesion composition in the form of a water-insoluble gel, membrane, foam or fiber, which is produced by combining anionic polysaccharides such as hyaluronic acid (HA) and carboxymethyl cellulose (CMC) with a hydrophobic polymer of polyglycolide. However, carboxymethyl cellulose is not a bio-derived material, and thus may cause a foreign body reaction or an inflammatory reaction *in vivo.* Meanwhile, polyglycolic acid (PGA) may release glycolic acid as a degradation product thereof to lower the surrounding acidity to an acidic pH that irritates the surrounding tissue and to cause an inflammatory reaction. In addition, when the composition is prepared in the form of a membrane, the material itself is inflexible and hard and thus brittle in a dry state, and the membrane rolls up upon contact with moisture, and when the composition is prepared in the form of a membrane, foam or fiber, it is inconvenient to handle because it is not fixed *in vivo* and may fold or cannot be adhered to a part requiring adhesion prevention.

Furthermore, US Patent No. 6,280,745 discloses a composition for drug delivery, which is used for the purpose of preventing adhesions. The composition includes one or more constitutive polymers such as a polyoxyalkylene block copolymer, a modifier polymer such as cellulose ethers, sodium carboxymethylcellulose and polyacrylates, and a co-surfactant such as fatty acid soap-based sodium oleate, sodium laurate, sodium caprate and sodium caprylate, and further includes those selected from various drugs including antibiotics, anti-inflammatory agents, and the like. Among the components of the composition, sodium carboxymethylcellulose (CMC-Na) is not a bio-derived material, is prepared by processing cellulose obtained from plants and is known to be able to cause a foreign body reaction *in vivo,* and other modifier polymers such as polyacrylates are not bio-derived materials, and thus have low biocompatibility, and also have problems in that a foreign body reaction may be caused.

Korean Patent No. 1330652 discloses an anti-adhesion composition which has an excellent anti-adhesion function by adding gelatin and chitosan to a PEG-PPG-PEG block copolymer and excellent adhesion performance so as to be continuously attached successfully to a wound site generated during surgery. However, such an anti-adhesion composition has a problem in terms of formulation stability in which chitosan is precipitated as the storage time passes, leading to a risk that an adhesion occurs.

PCT International Application published as WO 00/05974 A1 discloses liquid chitosan- containing compositions.

### [Related Art Documents]

### [Patent Documents]

1. Korean Patent No. 1082935
2. Korean Patent No. 1070358
3. US Patent No. 6,294,202
4. US Patent No. 6,280,745
5. Korean Patent No. 1330652
6. PCT International Application published as WO 00/05974 A1

### [Disclosure]

### [Technical Problem]

An object of the present invention is to provide an anti-adhesion polymer composition that has excellent adhesion performance so as to be continuously attached successfully to a wound site generated during surgery while also effectively exhibiting an anti-adhesion function, has antibacterial and hemostatic properties, and has excellent formulation stability.

Another object of the present invention is to provide an anti-adhesion polymer composition that is suitable for minimally invasive surgery, laparoscopic surgery, and the like, which are current surgical trends.

Still another object of the present invention is to provide an anti-adhesion polymer composition that has excellent biocompatibility by suppressing inflammatory reactions or foreign body reactions *in vivo* as much as possible.

### [Technical Solution]

The present invention provides an anti-adhesion polymer composition including: 24 to 50 wt% of a polyethylene glycol-polypropylene glycol-polyethylene glycol (PEG-PPG-PEG) block copolymer represented by the following Chemical Formula 1; 0.03 to 5 wt% of gelatin; 0.03 to 5 wt% of chitosan; and 0.01 to 1 wt% of an acid solution, wherein the anti-adhesion polymer composition has a pH of 3.5 or more.

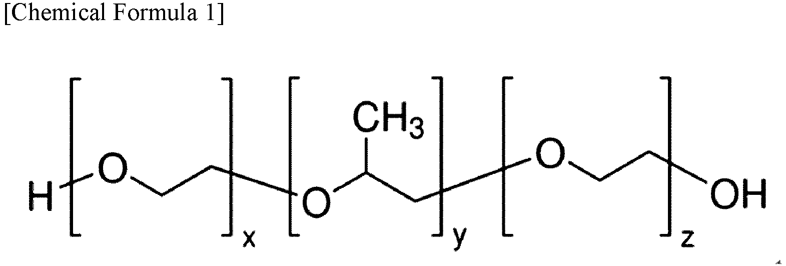

In Chemical Formula 1, x and z are each independently an integer from 75 to 110, y is an integer from 20 to 70, and the PEG-PPG-PEG block copolymer has a molecular weight of 6,000 to 20,000 Da.

Further, the present invention provides a method for preparing an anti-adhesion polymer composition, the method including: adding 0.03 to 5 wt% of gelatin, 0.03 to 5 wt% of chitosan and 0.01 to 1 wt% of an acid solution to distilled water and stirring the resulting mixture at 50 to 70°C for 10 to 60 minutes; and
lowering the temperature to 3 to 7°C, and then adding 24 to 50 wt% of a polyethylene glycol-polypropylene glycol-polyethylene glycol (PEG-PPG-PEG) block copolymer represented by Chemical Formula 1 thereto and stirring the resulting mixture for 1 to 10 hours.

### [Advantageous Effects]

The anti-adhesion polymer composition according to the present invention can be injected in a solution state and applied to a wound site caused by surgery, and laparoscopic and endoscopic surgery and gelled by body temperature to exhibit a function of suppressing adhesions of the wound site. In addition, the composition of the present invention can include an acid solution to more improve the formulation stability of the composition.

### [Description of Drawings]

FIG 1 illustrates the results of measuring chitosan stabilization according to acid addition.
FIG 2 illustrates the results of evaluating the anti-adhesion effect of the anti-adhesion polymer composition of the present invention.

### [Modes of the Invention]

The present invention relates to an anti-adhesion polymer composition including: 24 to 50 wt% of a polyethylene glycol-polypropylene glycol-polyethylene glycol (PEG-PPG-PEG) block copolymer represented by Chemical Formula 1; 0.03 to 5 wt% of gelatin; 0.03 to 5 wt% of chitosan; and
0.01 to 1 wt% of an acid solution, wherein the anti-adhesion polymer composition has a pH of 3.5 or more.

Hereinafter, the configuration of the present invention will be specifically described.

The anti-adhesion polymer composition according to the present invention can be injected in a solution state and applied to a wound site caused by surgery, and laparoscopic and endoscopic surgery and gelled by body temperature to exhibit a function of suppressing adhesions of the wound site.

In the present invention, the polyethylene glycol-polypropylene glycol-polyethylene glycol (PEG-PPG-PEG) block copolymer is a temperature-sensitive material that is present in a solution state at low temperature, but is gelled when the temperature rises. In the present invention, a PEG-PPG-PEG block copolymer may be used to prepare an anti-adhesion polymer composition that is harmless to the human body, is easy to use utilizing its temperature-sensitive characteristics, and has enhanced anti-adhesion effects.

In the present invention, the PEG-PPG-PEG block copolymer is represented by the following Chemical Formula 1.

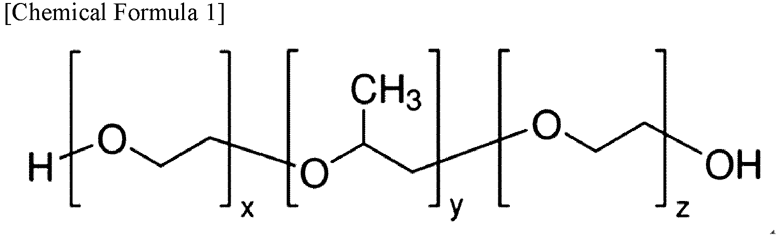

According to the invention, x and z in Chemical Formula 1 are each independently an integer from 75 to 110, and y is an integer from 20 to 70.
Further, the content of polyethylene glycol (PEG) in the PEG-PPG-PEG block copolymer may be 65 to 85 wt%, 68 to 82 wt% or 70 to 80 wt%. The PEG-PPG-PEG block copolymer may affect gelation and physical properties according to the ratio or molecular weight of PEG and PPG moieties. In particular, the content of PEG is an important factor that determines the hydrophilicity of the formulation, and when the content is too low, there is a risk that it may become difficult to mix the formulation. Therefore, it is desirable to adjust x, y and z within the above-described ranges.

In addition, the PEG-PPG-PEG block copolymer may have a molecular weight of 6,000 to 20,000 Da, or 8,000 to 10,000 Da. Since the gelation temperature and miscibility of the PEG-PPG-PEG block copolymer are affected by the molecular weight, the higher the molecular weight, the higher the gelation temperature and the lower the miscibility during composition preparation. Therefore, in the present invention, the formulation of the anti-adhesion polymer composition may be optimized by adjusting the molecular weight of the PEG-PPG-PEG block copolymer to 6,000 to 20,000 Da.

In an exemplary embodiment, the PEG-PPG-PEG block copolymer may be a mixture of two or more PEG-PPG-PEG block copolymers, and may specifically be a mixture of two PEG-PPG-PEG block copolymers.

When two PEG-PPG-PEG block copolymers are used, in one PEG-PPG-PEG block copolymer, x and z are each independently an integer from 90 to 110, and y may be an integer from 40 to 70. Furthermore, in the other PEG-PPG-PEG block copolymer, x and z are each independently an integer from 75 to 90, and y may be an integer from 20 to 40.

In an exemplary embodiment, as the PEG-PPG-PEG block copolymer, a polymer produced by BASF in Germany may be used, and specifically, commercially available trade names 'Pluronic' or 'Poloxamer' and the like may be used. PEG-PPG-PEG block copolymers of 'Pluronic' or 'Poloxamer' are divided into various types according to the ratio of PEG and PPG, and in the present invention, two or more of the above products may be mixed and used.

In the exemplary embodiments of the present invention, Poloxamer 407 and Poloxamer 188 were used as PEG-PPG-PEG block copolymers. Poloxamer 407 has a polyoxypropylene molecular mass of 4,000 Da and a PEG content of 70%. Further, Poloxamer 188 has a polyoxypropylene molecular mass of 1,800 Da and a PEG content of 80%. In the present invention, an anti-adhesion polymer composition having an excellent anti-adhesion effect may be prepared by mixing the above two components.

In an exemplary embodiment, the content of the PEG-PPG-PEG block copolymer is not particularly limited, and may be 24 to 50 wt% with respect to the total weight of the composition. When two PEG-PPG-PEG block copolymers are used, the content of one PEG-PPG-PEG block copolymer and the content of the other PEG-PPG-PEG block copolymer may each be 12 to 25 wt%. When the content of the PEG-PPG-PEG block copolymer is too low, it is difficult to form a gel even when the temperature is increased, and when the content is too high, the block copolymer is gelled even at low temperature, so that the content needs to be appropriately controlled. That is, in the above content range, the block copolymer may exhibit preferred gelation properties.

In the present invention, gelatin and chitosan are bio-derived polymers and form bonds between PEG-PPG-PEG block copolymers by hydrogen bonds between O²⁻ --- H⁺ and N³⁻ --- H⁺ ions and/or ionic bonds between NH³⁺ ---OH⁻ functional groups. Such a bio-derived polymer improves the adhesion performance of the polymer composition to fix the composition to a wound site and prevents the composition from moving to other sites, thereby enhancing the effect of preventing adhesion of the wound site.

In the present invention, gelatin has the property of absorbing water and being swollen. In addition, the intestinal surface is known to generally have a negative charge, and in contrast, gelatin, which is a polymer that adheres to the intestinal surface, has a positive charge as a cationic group, so electrostatic bonds may be produced between the intestinal surface and gelatin to maintain adhesion, resulting in excellent adhesion to the intestinal surface, that is, bioadhesive properties. Furthermore, since gelatin increases the osmotic pressure of the wound site and promotes platelet activation (TISSUE ENGINEERING, Volume 11, Number 7/8, 2005), the polymer composition of the present invention containing gelatin has hemostatic properties that promote blood coagulation.

In an exemplary embodiment, gelatin may have a 200 to 300 bloom.

Meanwhile, chitosan is a polymer that belongs to the cationic group among hydrogel-type intestinal adhesive polymers and is generally positively charged, so chitosan can form electrostatic bonds with the intestinal surface, and can adhere to the intestinal surface while remaining on the intestinal surface according to the electronic theory of intestinal surface adhesion. Therefore, the chitosan may help the adhesion of gelatin to the intestinal surface to further improve the adhesion. Further, chitosan imparts an additional antibacterial effect through a -NH₃ functional group (J. Ocul. Pharmacol. Ther. 16 (2000) 261270) (J. Appl. Polym. Sci. 79 (2001) 13241335), and thus may allow the polymer composition of the present invention to exhibit excellent antibacterial characteristics.

In an exemplary embodiment, the degree of deacetylation of chitosan may be 70 to 90%.

According to the invention, the content of gelatin is 0.03 to 5 wt%.

According to the invention, the content of chitosan is 0.03 to 5 wt%. When the content of gelatin and/or chitosan is low, it is difficult to substantially expect the effect of adding these components. In addition, when the content of gelatin and/or chitosan is high, the content may be too high, so gelatin and or chitosan may remain undissolved in the polymer composition, and when the composition is stored for a long period of time (about 3 months), chitosan and gelatin may separate from each other in the composition, which is undesirable in terms of long-term stability. That is, within the above-described content ranges of gelatin and chitosan, the anti-adhesion polymer composition may exhibit preferred gelation properties at body temperature and may impart biocompatibility.

Furthermore, the anti-adhesion polymer composition of the present invention includes an acid solution. The acid solution may increase the solubility of chitosan and improve the formulation stability of the anti-adhesion polymer composition.

In an exemplary embodiment, the type of acid is not particularly limited, and may be one or more selected from the group consisting of hydrochloric acid, acetic acid, ascorbic acid, nitric acid, sulfuric acid, hydrofluoric acid, phosphoric acid, citric acid, lactic acid and formic acid.

In an exemplary embodiment, a high purity acid with a concentration of 99.9% or 100% may be used as the acid solution. Although it is possible to dilute the above acids and use them, a high purity acid was used in the examples of the present invention.

According to the invention, the content of the acid solution is 0.01 to 1 wt% with respect to the total weight of the anti-adhesion polymer composition. Within the above content range, the solubility of chitosan is excellent, and when the content exceeds 1 wt%, the pH of the composition is low, so there is a risk that side effects may occur when the composition is applied to the body.

The anti-adhesion polymer composition according to the present invention may further include a growth factor in addition to the above-described components.

In an exemplary embodiment, the type of growth factor is not particularly limited, and it is possible to use one or more selected from the group consisting of epidermal growth factors (EGFs, beta-urogastrone), a heparin-binding EGF-like growth factor (HB-EGF), a transforming growth factor-α (TGF-α) and fibroblast growth factors (FGFs) which may promote the tissue regeneration of the wound site.

In an exemplary embodiment, the content of growth factor is not particularly limited, but from the viewpoint of bio-homeostasis and reduction of side effects, 1 µg/ml to 1 mg/ml is suitable based on the results of previous animal experiments.

In addition, the anti-adhesion polymer composition according to the present invention may further include a stabilizer for suppressing the phase separation of the polymer composition, in addition to the above-described components. The stabilizer may be one or more selected from the group consisting of glycerol, glycerides and glycols.

In an exemplary embodiment, the stabilizer may be used in an amount of 1 to 20 wt%.

The anti-adhesion polymer composition according to the present invention has a pH of 3.5 or more. The pH may be 4.0 or more or 4.1 or more, and the upper limit thereof may be 6.0 or less. The pH of blood is 7.4, and the pH in the body is 7.3 to 7.75, which is slightly alkaline. Therefore, there is a risk that upon contact with a strongly acidic product, side effects due to the pH in the body occur. In the present invention, the anti-adhesion polymer composition having a pH of 3.5 or more may be used to have excellent anti-adhesion properties without affecting organs when used in the body.

Furthermore, the anti-adhesion polymer composition according to the present invention may have a viscosity of 1,500 to 5,000 cPs at 25°C and 15,000 to 50,000 cPs at 27°C. The viscosity means a result measured using a Brookfield viscometer. Within the above viscosity range, the anti-adhesion polymer composition is easily attached to organs such as the intestines and may be easily degraded. In particular, when the composition has a viscosity of 15,000 cPs or less at 37°C, there is a risk that flowability occurs and the adhesion effect is reduced. Further, when the composition includes a growth factor having a viscosity within the above range, the growth factor may be easily released. When the viscosity is too low, the ability to adhere to the intestines deteriorates, and when the viscosity is too high, the composition may cause an adhesion, so it is desirable to adjust the viscosity within the above range.

In addition, the present invention relates to a method for preparing the above-described anti-adhesion polymer composition.

The anti-adhesion polymer composition may be prepared by (S1) adding 0.03 to 5 wt% of gelatin, 0.03 to 5 wt% of chitosan and 0.01 to 1 wt% of an acid solution to distilled water and stirring the resulting mixture at 50 to 70°C for 10 to 60 minutes; and
(S2) lowering the temperature to 3 to 7°C, and then adding 24 to 50 wt% of a polyethylene glycol-polypropylene glycol-polyethylene glycol (PEG-PPG-PEG) block copolymer represented by Chemical Formula 1 thereto and stirring the resulting mixture for 1 to 10 hours.

Step (S1) is a step of adding gelatin and chitosan to distilled water and stirring the resulting mixture.

In an exemplary embodiment, as gelatin and chitosan, the above-described gelatin and chitosan may be used, and the content thereof may each be 0.03 to 5 wt% with respect to the total weight (100 wt%) of the components used during the preparation of the anti-adhesion polymer composition.

In an exemplary embodiment, the step may be performed at 50 to 70°C for 10 to 60 minutes.

In an exemplary embodiment, the step may be performed by stirring at 100 to 300 rpm in a hot water bath.

Step (S2) is a step of lowering the temperature to 3 to 7°C, and then adding a PEG-PPG-PEG block copolymer represented by Chemical Formula 1 thereto and stirring the resulting mixture. Through the steps, the anti-adhesion polymer composition according to the present invention may be prepared.

In an exemplary embodiment, adding a stabilizer before performing Step (S2) may be further included. As the stabilizer, the above-described type of stabilizer may be used, and glycerin may be used in the present invention.

In an exemplary embodiment, the temperature may be slowly lowered to 3 to 7°C in the step.

In an exemplary embodiment, the above-described PEG-PPG-PEG block copolymer may be used as the PEG-PPG-PEG block copolymer in the step, and the content thereof may be 24 to 50 wt% with respect to the total weight (100 wt%) of the components used during the preparation of the anti-adhesion polymer composition. In particular, two PEG-PPG-PEG block copolymers may be used, and in this case, the content of one PEG-PPG-PEG block copolymer and the content of the other PEG-PPG-PEG block copolymer may each be 12 to 25 wt%.

In an exemplary embodiment, the step may be performed under stirring for 1 to 10 hours.

In an exemplary embodiment, the anti-adhesion polymer composition according to the present invention may be composed by further performing a step of adding a growth factor during the preparation process, or including a growth factor before being applied to a wound site. In this case, the composition may be used after injecting a growth factor by an in situ syringe and mixing the resulting mixture before application to a wound site. The anti-adhesion polymer composition according to the present invention forms a membrane by body temperature so as to prevent an adhesion from occurring at the wound site, and may be gelled to release the growth factor.

Described but not claimed is a method for applying the above-described anti-adhesion polymer composition.

The anti-adhesion polymer composition according to the present invention may be applied through first, a step of injecting the polymer composition in a solution state into a wound site during or after surgery, second, a step in which the polymer composition is fixed to the wound site by a bio-derived polymer included in the polymer composition, and third, a step in which the polymer composition in a solution state is gelled while the temperature rises by body temperature.

The anti-adhesion polymer composition of the present invention is present in a solution state at room temperature due to the temperature sensitivity of a PEG-PPG-PEG block copolymer chemically bonded to an appropriate composition, and when applied to the body, the composition is gelled by a temperature of 37°C and acts as a barrier to prevent adhesions at a wound tissue site. Since the composition is present in the form of a solution at room temperature, the composition may be injected into the wound site, and after being applied, the composition may be gelled and concentrated locally on the wound site, thereby improving the barrier effect against adhesions.

After the anti-adhesion polymer composition is gelled, the composition may be degraded over time by in vivo degrading enzymes and metabolism and excreted.

Hereinafter, the present invention will be described in detail through the Examples. However, the following examples are only for exemplifying the present invention, and the scope of the present invention is not limited to the following Examples. The present examples are provided to make the disclosure of the present invention complete and to allow a person skilled in the art to which the present invention belongs to completely comprehend the scope of the present invention, and the present invention is defined only by the scope of the claims.

### [Mode for Invention]

### Examples

### Comparative Example 1.

0.05 wt% of gelatin and 0.3 wt% of chitosan were added to sterilized distilled water, and the resulting mixture was stirred at 150 rpm in a hot water bath at 55 to 60°C for 30 minutes. Thereafter, 10 wt% of glycerin was added thereto, and the mixture was stirred under the same conditions while slowly lowering the temperature to 4 to 6°C. When the temperature was lowered, 17% by weight of Poloxamer 188 and 15 wt% of Poloxamer 407 were added thereto, and the resulting mixture was stirred at 100 rpm for 3 hours to prepare an anti-adhesion polymer composition.

### Example 1.

0.05 wt% of gelatin, 0.3 wt% of chitosan and 0.01 wt% of 100% acetic acid were added to sterilized distilled water, and the resulting mixture was stirred at 150 rpm in a hot water bath at 55 to 60°C for 30 minutes. 10 wt% of glycerin was added thereto, and the mixture was stirred under the same conditions while slowly lowering the temperature to 4 to 6°C. When the temperature was lowered, 17% by weight of Poloxamer 188 and 15 wt% of Poloxamer 407 were added thereto, and the resulting mixture was stirred at 100 rpm for 3 hours to prepare an anti-adhesion polymer composition.

### Example 2.

An anti-adhesion polymer composition was prepared in the same manner as in Example 1, except that 0.1 wt% of acetic acid was used instead of 0.01 wt% of acetic acid.

### Example 3.

An anti-adhesion polymer composition was prepared in the same manner as in Example 1, except that 0.5 wt% of acetic acid was used instead of 0.01 wt% of acetic acid.

### Example 4.

An anti-adhesion polymer composition was prepared in the same manner as in Example 1, except that 1 wt% of acetic acid was used instead of 0.01 wt% of acetic acid.

### Comparative Example 2.

An anti-adhesion polymer composition was prepared in the same manner as in Example 1, except that 3 wt% of acetic acid was used instead of 0.01 wt% of acetic acid.

### Example 5.

An anti-adhesion polymer composition was prepared in the same manner as in Example 1, except that 0.01 wt% of 100% hydrochloric acid was used instead of 0.01 wt% of acetic acid.

### Example 6.

An anti-adhesion polymer composition was prepared in the same manner as in Example 1, except that 0.01 wt% of 100% phosphoric acid was used instead of 0.01 wt% of acetic acid.

### Example 7.

An anti-adhesion polymer composition was prepared in the same manner as in Example 1, except that 0.01 wt% of 100% citric acid was used instead of 0.01 wt% of acetic acid.

### Example 8.

An anti-adhesion polymer composition was prepared in the same manner as in Example 1, except that 0.01 wt% of 100% lactic acid was used instead of 0.01 wt% of acetic acid.

### Experimental Example 1. Effect of chitosan stabilization according to acid addition

Chitosan stabilization according to acid addition was evaluated by a method of observing whether chitosan was precipitated by storing the anti-adhesion polymer compositions prepared in the Examples and Comparative Examples at 4°C, 25°C and 40°C for 1 day, 7 days, 28 days and 60 days.

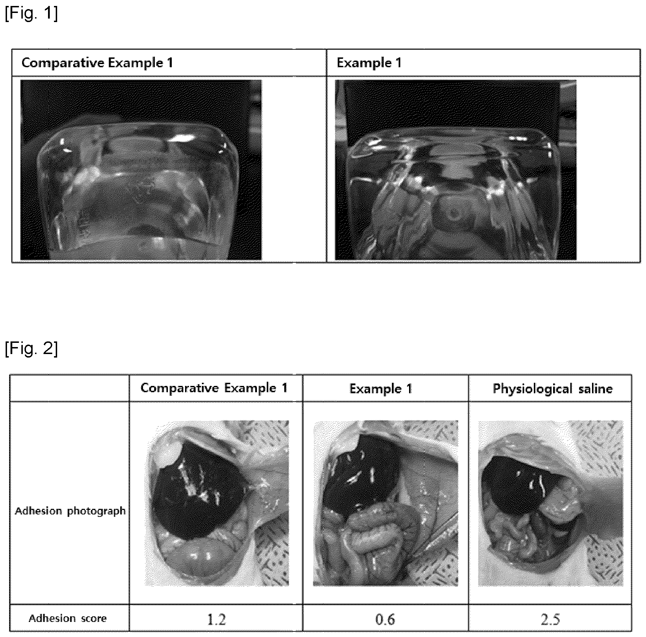

The evaluation results are shown in the following Table 1 and FIG 1.

**[Table 1]**

| Example | Precipitation | | |
|---|---|---|---|
| | 4°C | 25°C | 40°C |
| Comparative Example 1 | Present (7 days) | Present (28 days) | Present (28 days) |
| Example 1 | Absent | Absent | Absent |
| Example 2 | Absent | Absent | Absent |
| Example 3 | Absent | Absent | Absent |
| Example 4 | Absent | Absent | Absent |
| Comparative Example 2 | Absent | Absent | Absent |
| Example 5 | Absent | Absent | Absent |
| Example 6 | Absent | Absent | Absent |
| Example 7 | Absent | Absent | Absent |
| Example 8 | Absent | Absent | Absent |

As illustrated in Table 1 and FIG 1, it can be confirmed that even though water-soluble chitosan is used, when the chitosan is dissolved while being mixed with other materials, chitosan is precipitated without being stabilized. When chitosan was precipitated, the precipitation phenomenon appeared within a minimum of 7 days and a maximum of 28 days (Comparative Example 1).

From the above results, it can be confirmed that the solubility of chitosan is increased and the formulation is stabilized by adding an acid to the anti-adhesion polymer composition according to the present invention.

### Experimental Example 2. Effect of viscosity according to type of acid

It was confirmed whether viscosity, which is the characteristic of a product, was affected by the addition of various acids.

Specifically, the change in viscosity of the anti-adhesion polymer compositions prepared in the Examples and Comparative Examples according to temperature was measured using a Brookfield viscometer.

Measured viscosities are shown in the following Table 2.

**[Table 2]**

| | Viscosity (cPs) | |
|---|---|---|
| | 25°C | 37°C |
| Comparative Example 1 | 1916 | 27097 |
| Example 1 | 2366 | 27660 |
| Example 2 | 2020 | 18992 |
| Example 3 | 2195 | 16770 |
| Example 4 | 2897 | 25647 |
| Comparative Example 2 | 2241 | 18252 |
| Example 5 | 4785 | 45697 |
| Example 6 | 3061 | 35284 |
| Example 7 | 2879 | 23967 |
| Example 8 | 3294 | 26579 |

As shown in Table 2, it can be seen that the viscosity is not significantly affected by the acid content, and the anti-adhesion polymer composition has flowability at room temperature and forms a highly viscous gel at body temperature. Therefore, it can be expected that the anti-adhesion polymer composition can be attached inside the body.

### Experimental Example 3. Measurement of pH according to acid content

As an acid is added to the anti-adhesion polymer composition, the pH of each of the anti-adhesion polymer compositions prepared in the Examples and Comparative Examples was measured in order to confirm the effect on the pH of the material and set a pH range suitable for use in the body.

The measurement results are shown in the following table.

**[Table 3]**

| | pH |
|---|---|
| Comparative Example 1 | 4.54 |
| Example 1 | 4.49 |
| Example 2 | 4.21 |
| Example 3 | 4.05 |
| Example 4 | 3.72 |
| Comparative Example 2 | 3.36 |
| Example 5 | 3.92 |
| Example 6 | 3.66 |
| Example 7 | 4.02 |
| Example 8 | 4.10 |

The pH of blood is 7.4, and the pH in the body is 7.3 to 7.75, which is slightly alkaline. Therefore, upon contact with a strongly acidic product, side effects due to the pH in the body may occur.

It can be confirmed that the anti-adhesion polymer composition of the present Example has a pH of 3.5 or more and is suitable for use in the body. Since Comparative Example 2 including 3 wt% of the acid solution has a pH of less than 3.5, there is a risk that side effects may occur when Comparative Example 2 is applied to the body.

### Experimental Example 4. Measurement of stress

It was confirmed whether the attachment performance of the anti-adhesion polymer composition was affected by the addition of various acids.

Specifically, stress was measured for the anti-adhesion polymer compositions prepared in the Examples and Comparative Examples, and the stresses between Example 1 of the present invention and the composition (Example 1) of Korean Patent No. 1330652 were compared.

After a sample including 1 mL of each composition was applied to a slide glass and placed in an incubator at 37°C for about 10 minutes to gel the sample, the slide glass with the sample applied was fixed in a universal testing machine, the polymer composition and the surface of an adapter were adhered, and then the maximum stress when the polymer composition and the adapter were adhered and separated was measured.

The measurement results are shown in the following Table 4.

**[Table 4]**

| Sample | Maximum stress (Mpa) |
|---|---|
| Composition of Korean Patent No. 1330652 | 0.03 |
| Example 1 | 0.03 |

As shown in Table 4, it can be confirmed that there is no difference in maximum stress between the composition of Korean Patent No. 1330652 and the anti-adhesion polymer composition of Example 1 of the present invention. Through this, it can be confirmed that the addition of an acid does not affect the adhesion ability of the anti-adhesion polymer composition.

### Experimental Example 5. Anti-adhesion test

The surface of the cecum and the peritoneum of rats were artificially injured to induce forced adhesions, and wound sites were treated with the anti-adhesion polymer compositions of the present invention prepared according to Comparative Example 1 and Example 1, and then the anti-adhesion effect of the rat cecum and peritoneum was observed one week later. The degree of adhesion was evaluated by a scale from 0 to 5 (Score indicating the degree of adhesion; 0 = the case where no adhesion occurs, 1 = the case where a focal adhesion occurs, 2 - the case where there are many focal adhesions, 3 = the case where a surface adhesion occurs, 4 = the case where a surface adhesion is deep, and 5 = the case where vessels are formed with a surface adhesion), and the results are illustrated in FIG 2.

As illustrated in FIG 2, it can be confirmed that an adhesion occurred in a physiological saline group, and Comparative Example 1 exhibited a higher adhesion rate than Example 1. Through this, it can be confirmed that, by the addition of an acid, the stability of the formulation is maintained and the anti-adhesion effect is affected.

### [Industrial Applicability]

The anti-adhesion polymer composition according to the present invention can be injected in a solution state and applied to a wound site caused by surgery, and laparoscopic and endoscopic surgery and gelled by body temperature to exhibit a function of suppressing an adhesion of the wound site. In addition, the composition of the present invention includes an acid solution to further improve the formulation stability of the composition.

## Claims

1. An anti-adhesion polymer composition comprising: 24 to 50 wt% of a polyethylene glycol-polypropylene glycol-polyethylene glycol (PEG-PPG-PEG) block copolymer represented by the following Chemical Formula 1; 0.03 to 5 wt% of gelatin; 0.03 to 5 wt% of chitosan; and 0.01 to 1 wt% of an acid solution, wherein the anti-adhesion polymer composition has a pH of 3.5 or more: 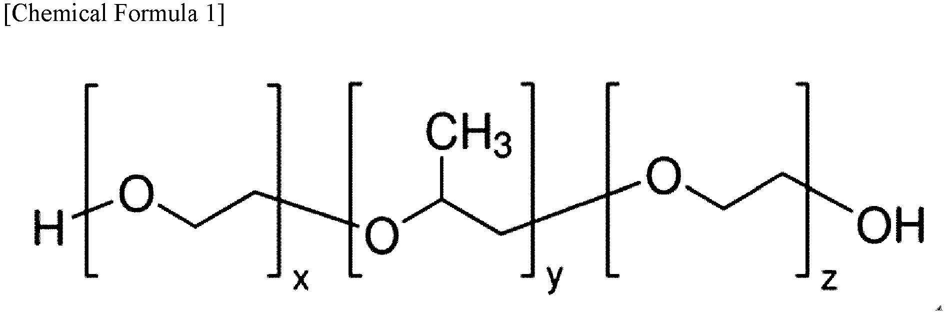 wherein, in Chemical Formula 1, x and z are each independently an integer from 75 to 110, y is an integer from 20 to 70, and the PEG-PPG-PEG block copolymer has a molecular weight of 6,000 to 20,000 Da.

2. The anti-adhesion polymer composition of claim 1, wherein the PEG-PPG-PEG block copolymer is a mixture of two or more PEG-PPG-PEG block copolymers.

3. The anti-adhesion polymer composition of claim 2, wherein when two PEG-PPG-PEG block copolymers are used,
x and z are each independently an integer from 90 to 110, and y is an integer from 40 to 70 in one PEG-PPG-PEG block copolymer, and
x and z are each independently an integer from 75 to 90, and y is an integer from 20 to 40 in the other PEG-PPG-PEG block copolymer.

4. The anti-adhesion polymer composition of claim 2, wherein when two PEG-PPG-PEG block copolymers are used, the content of one PEG-PPG-PEG block copolymer and the content of the other PEG-PPG-PEG block copolymer are each 12 to 25 wt%.

5. The anti-adhesion polymer composition of claim 1, wherein the acid in the acid solution comprises one or more selected from the group consisting of hydrochloric acid, acetic acid, ascorbic acid, nitric acid, sulfuric acid, hydrofluoric acid, phosphoric acid, citric acid, lactic acid and formic acid.

6. The anti-adhesion polymer composition of claim 1, further comprising a growth factor,
wherein the growth factor comprises one or more selected from the group consisting of epidermal growth factors (EGFs, beta-urogastrone), a heparin-binding EGF-like growth factor (HB-EGF), a transforming growth factor-α (TGF-α) and fibroblast growth factors (FGFs).

7. The anti-adhesion polymer composition of claim 1, further comprising a stabilizer,
wherein the stabilizer comprises one or more selected from the group consisting of glycerol, glycerides and glycols.

8. A method for preparing an anti-adhesion polymer composition of claim 1, the method comprising: adding 0.03 to 5 wt% of gelatin, 0.03 to 5 wt% of chitosan and 0.01 to 1 wt% of an acid solution to distilled water and stirring the resulting mixture at 50 to 70°C for 10 to 60 minutes; and
lowering the temperature to 3 to 7°C, and then adding 24 to 50 wt% of a polyethylene glycol-polypropylene glycol-polyethylene glycol (PEG-PPG-PEG) block copolymer represented by the following Chemical Formula 1 thereto and stirring the resulting mixture for 1 to 10 hours: 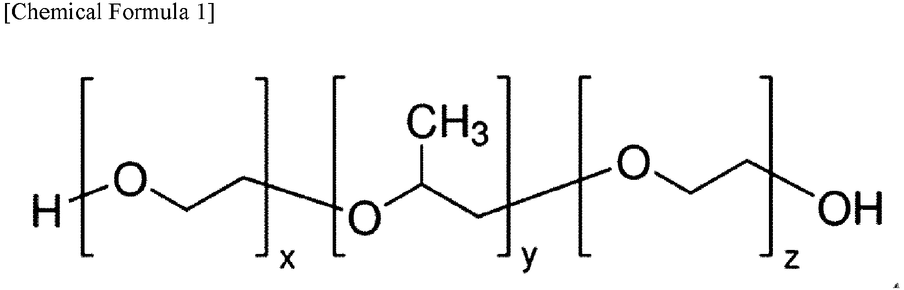
wherein, in Chemical Formula 1, x and z are each independently an integer from 75 to 110, y is an integer from 20 to 70, and the PEG-PPG-PEG block copolymer has a molecular weight of 6,000 to 20,000 Da.

## Patentansprüche

1. Anti-Adhäsions-Polymerzusammensetzung, umfassend: 24 bis 50 Gew.-% eines Polyethylenglykol-Polypropylenglykol-Polyethylenglykol (PEG-PPG-PEG)-Blockcopolymers, dargestellt durch die folgende chemische Formel 1; 0,03 bis 5 Gew.-% Gelatine; 0,03 bis 5 Gew.-% Chitosan; und 0,01 bis 1 Gew.-% einer Säurelösung, wobei die Anti-Adhäsions-Polymerzusammensetzung einen pH-Wert von 3,5 oder mehr aufweist: 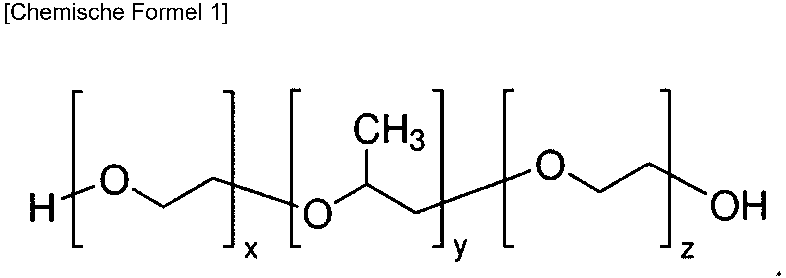 wobei in der chemischen Formel 1 x und z jeweils unabhängig voneinander eine ganze Zahl von 75 bis 110 sind, y eine ganze Zahl von 20 bis 70 ist und das PEG-PPG-PEG-Blockcopolymer ein Molekulargewicht von 6.000 bis 20.000 Da hat.

2. Anti-Adhäsions-Polymerzusammensetzung nach Anspruch 1, wobei das PEG-PPG-PEG-Blockcopolymer eine Mischung aus zwei oder mehr PEG-PPG-PEG-Blockcopolymeren ist.

3. Anti-Adhäsions-Polymerzusammensetzung nach Anspruch 2, wobei, wenn zwei PEG-PPG-PEG Blockcopolymere verwendet werden,
x und z jeweils unabhängig voneinander eine ganze Zahl von 90 bis 110 sind und y eine ganze Zahl von 40 bis 70 in einem PEG-PPG-PEG-Blockcopolymer ist, und
x und z jeweils unabhängig voneinander eine ganze Zahl von 75 bis 90 sind und y eine ganze Zahl von 20 bis 40 in dem anderen PEG-PPG-PEG-Blockcopolymer ist.

4. Anti-Adhäsions-Polymerzusammensetzung nach Anspruch 2, wobei, wenn zwei PEG-PPG-PEG Blockcopolymere verwendet werden, der Gehalt eines PEG-PPG-PEG-Blockcopolymers und der Gehalt des anderen PEG-PPG-PEG-Blockcopolymers jeweils 12 bis 25 Gew.-% betragen.

5. Anti-Adhäsions-Polymerzusammensetzung nach Anspruch 1, wobei die Säure in der Säurelösung eine oder mehrere, ausgewählt aus der Gruppe bestehend aus Salzsäure, Essigsäure, Ascorbinsäure, Salpetersäure, Schwefelsäure, Flusssäure, Phosphorsäure, Zitronensäure, Milchsäure und Ameisensäure, umfasst.

6. Anti-Adhäsions-Polymerzusammensetzung nach Anspruch 1, weiter umfassend einen Wachstumsfaktor,
wobei der Wachstumsfaktor einen oder mehrere, ausgewählt aus der Gruppe bestehend aus epidermalen Wachstumsfaktoren (EGFs, Beta-Urogastron), einem Heparinbindenden EGF-ähnlichen Wachstumsfaktor (HB-EGF), einem transformierenden Wachstumsfaktor-α (TGF-α) und Fibroblasten-Wachstumsfaktoren (FGFs), umfasst.

7. Anti-Adhäsions-Polymerzusammensetzung nach Anspruch 1, weiter umfassend einen Stabilisator, wobei der Stabilisator einen oder mehrere, ausgewählt aus der Gruppe bestehend aus Glycerin, Glyceriden und Glykolen, umfasst.

8. Verfahren zur Herstellung einer Anti-Adhäsions-Polymerzusammensetzung nach Anspruch 1, wobei das Verfahren Folgendes umfasst: Hinzufügen von 0,03 bis 5 Gew.-% Gelatine, 0,03 bis 5 Gew.-% Chitosan und 0,01 bis 1 Gew.-% einer Säurelösung zu destilliertem Wasser und Rühren der resultierenden Mischung bei 50 bis 70 °C für 10 bis 60 Minuten; und
Absenken der Temperatur auf 3 bis 7 °C, und dann Hinzufügen von 24 bis 50 Gew.-% eines Polyethylenglykol-Polypropylenglykol-Polyethylenglykol (PEG-PPG-PEG)-Blockcopolymers, dargestellt durch die folgende chemische Formel 1, und Rühren der resultierenden Mischung für 1 bis 10 Stunden: 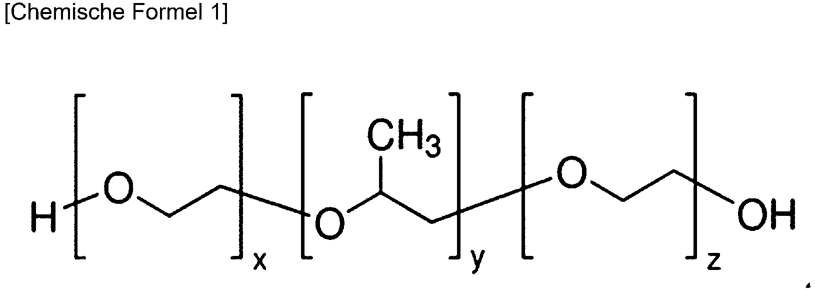
wobei in der chemischen Formel 1 x und z jeweils unabhängig voneinander eine ganze Zahl von 75 bis 110 sind, y eine ganze Zahl von 20 bis 70 ist und das PEG-PPG-PEG-Blockcopolymer ein Molekulargewicht von 6.000 bis 20.000 Da hat.

## Revendications

1. Composition polymère anti-adhérence comprenant : 24 à 50 % en poids d'un copolymère à blocs polyéthylène glycol-polypropylène glycol-polyéthylène glycol (PEG-PPG-PEG) représenté par la formule chimique 1 suivante ; 0,03 à 5 % en poids de gélatine ; 0,03 à 5 % en poids de chitosane ; et 0,01 à 1 % en poids d'une solution acide, dans laquelle la composition polymère anti-adhérence a un pH de 3,5 ou plus : 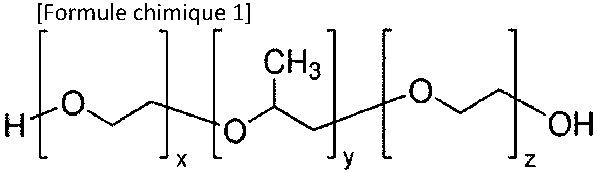 dans laquelle, dans la formule chimique 1, x et z sont chacun indépendamment un entier de 75 à 110, y est un entier de 20 à 70, et le copolymère à blocs PEG-PPG-PEG a un poids moléculaire de 6 000 à 20 000 Da.

2. Composition polymère anti-adhérence selon la revendication 1, dans laquelle le copolymère à blocs PEG-PPG-PEG est un mélange de deux copolymères en blocs PEG-PPG-PEG ou plus.

3. Composition polymère anti-adhérence selon la revendication 2, dans laquelle lorsque deux copolymères à blocs PEG-PPG-PEG sont utilisés,
x et z sont chacun indépendamment un entier de 90 à 110, et y est un entier de 40 à 70 dans un copolymère à blocs PEG-PPG-PEG, et
x et z sont chacun indépendamment un entier de 75 à 90, et y est un entier de 20 à 40 dans l'autre copolymère à blocs PEG-PPG-PEG.

4. Composition polymère anti-adhérence selon la revendication 2, dans laquelle, lorsque deux copolymères à blocs PEG-PPG-PEG sont utilisés, la teneur d'un copolymère à blocs PEG-PPG-PEG et la teneur de l'autre copolymère à blocs PEG-PPG-PEG sont chacune de 12 à 25 % en poids.

5. Composition polymère anti-adhérence selon la revendication 1, dans laquelle l'acide dans la solution acide comprend un ou plusieurs sélectionnés dans le groupe constitué par l'acide chlorhydrique, l'acide acétique, l'acide ascorbique, l'acide nitrique, l'acide sulfurique, l'acide fluorhydrique, l'acide phosphorique, l'acide citrique, l'acide lactique et l'acide formique.

6. Composition polymère anti-adhérence selon la revendication 1, comprenant en outre un facteur de croissance,
dans laquelle le facteur de croissance comprend un ou plusieurs sélectionnés dans le groupe constitué par les facteurs de croissance épidermiques (EGF, bêta-urogastrone), un facteur de croissance de type EGF de liaison à l'héparine (HB-EGF), un facteur de croissance transformant-a (TGF-α) et les facteurs de croissance des fibroblastes (FGF).

7. Composition polymère anti-adhérence selon la revendication 1, comprenant en outre un stabilisant,
dans laquelle le stabilisant comprend un ou plusieurs sélectionnés dans le groupe constitué par le glycérol, les glycérides et les glycols.

8. Procédé pour préparer une composition polymère anti-adhérence selon la revendication 1, le procédé comprenant : l'ajout de 0,03 à 5 % en poids de gélatine, de 0,03 à 5 % en poids de chitosane et de 0,01 à 1 % en poids d'une solution acide à de l'eau distillée et l'agitation du mélange résultant à 50 à 70 °C pendant 10 à 60 minutes ; et
l'abaissement de la température à 3 à 7 °C, puis l'ajout de 24 à 50 % en poids d'un copolymère à blocs polyéthylène glycol-polypropylène glycol-polyéthylène glycol (PEG-PPG-PEG) représenté par la formule chimique 1 suivante et l'agitation du mélange résultant pendant 1 à 10 heures : 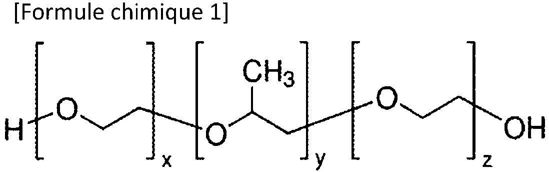
dans laquelle, dans la formule chimique 1, x et z sont chacun indépendamment un entier de 75 à 110, y est un entier de 20 à 70, et le copolymère à blocs PEG-PPG-PEG a un poids moléculaire de 6 000 à 20 000 Da.
